# EUROPÄISCHE PATENTANMELDUNG

(11) **EP 1 284 467 A2**
(43) Veröffentlichungstag der Anmeldung: **19.02.2003**
(21) Anmeldenummer: 02017098.1
(22) Anmeldetag: 30.07.2002
(51) Int. Cl.: G06F 19/00

(54) **Verfahren zur Erstellung und Auswertung einer medizinischen Datenbank**

(30) Priorität: 16.08.2001 DE 10140152
(71) Anmelder: Stähle, Kurt, 75242 Neuhausen-Steinegg (DE)
(72) Erfinder: Stähle, Kurt, 75242 Neuhausen-Steinegg (DE)
(74) Vertreter: Frank, Gerhard, Dipl.-Phys.

(57) **Zusammenfassung**

Zur Erfassung und statistischen Sicherung von Zusammenhängen zwischen der Iris eines Probanden und dessen Gesundheitszustand wird eine Datenbank mit Abbildungen der Iris von M Probanden erstellt, mit folgenden Verfahrensschritten:
Gewinnung einer Abbildung der Iris (Iriskarte) der M Probanden durch optischelektronisches Einscannen der Bildpunkte mit bestimmtem Farbwert in einem vorgebbaren Raster, Umsetzung und Abspeicherung der M eingescannten Iriskarten in M Dateien mit jeweils a x b Datenfeldern mit einem Wert entsprechend dem Farbwert des zugehörigen Bildpunkts, Normierung der M Dateien derart, dass für jedes Datenfeld jeweils ein durch eine Rechenvorschrift vorgebbarer Normierungswert gebildet wird, der den normalen Farbwert des zugehörigen Bildpunktes darstellt, Bildung einer Normdatei (DQ) aus den derart normierten Mittelwerten, die eine Iriskarte mit die dem statistischen Normalzustand der Iris der M Probanden darstellt und Abspeicherung der Normdatei (DQ) in einem ersten Abschnitt eines Datenträgers.

Die Normierung gestattet eine statistisch eindeutige Zuordnung (sofern vorhanden) zwischen spezifischen Krankheiten und entsprechenden Abweichungen der Irisabbildung von einem Normalzustand. Dies kann für eine computergesteuerte Irisdiagnose benutzt werden.

## Beschreibung

### Hintergrund der Erfindung

Die Erfindung betrifft ein Verfahren gemäß dem Oberbegriff des Patentanspruchs 1.

Es ist seit langem bekannt, dass der Zustand der Organe des menschlichen und tierischen Körpers sich in vom eigentlichen Krankheitsherd entfernten Körperbereichen wiederspiegelt, indem dort Veränderungen hervorgerufen werden, die ihrerseits einer Beobachtung oder Messung zugänglich sind. Einen typischen derartigen Indikator stellt die Iris dar. Die Iris wird von Augendiagnostikern zur Beurteilung des Gesundheitszustandes eines Patienten benutzt.

### Darstellung der Erfindung

Aufgabe der Erfindung ist die Erfassung und statistische Sicherung von Zusammenhängen zwischen der Iris eines Probanden und dessen Gesundheitszustand.

Der Grundgedanke der Erfindung besteht darin, dass eine Datenbank aufgebaut wird, in der eingescannte Irisabbildungen von Probanden eingespeichert werden, wobei diese Einspeicherung durch ein elektronisch-optisches Scannen beispielsweise zeilenweise erfolgt, wie dies auch beim Scannen von Fotografien, Bildern oder auch vom Scannen von Landschaftsabschnitten durch Satelliten an sich bekannt ist. Die Normierung gestattet eine statistisch eindeutige Zuordnung (sofern vorhanden) zwischen spezifischen Krankheiten und entsprechenden Abweichungen der Irisabbildung von einem Normalzustand. Dies kann für eine computergesteuerte Irisdiagnose benutzt werden.

Es wird also eine "Iriskarte" mit einer bestimmten Anzahl von Pixeln oder Datenfeldern erzeugt, in denen jeweils die zu einem Irissegment gehörigen eingescannte Information bezüglich Helligkeit und Farbe dieses Segments eingelesen wird. Daraus lässt sich eine "Norm-Iriskarte" bilden.

Ausser den insgesamt M Probanden mit "Normaliris" wird eine Anzahl M Probanden betrachtet, die ein konkretes Gesundheitsmerkmal aufweisen, beispielsweise einen bestimmten Organzustand, oder auch eine sonstige gesundheitliche Veränderung, die nach bisherigen Erfahrungen durch eine entsprechende Modifizierung eines oder mehrerer Segmente der "Normaliris" erkennbar wird.

Mittels eines geeigneten Rechenprogramms, beispielsweise durch Anwendung geeigneter Filter in Datenbankprogrammen, werden die Irisabbildungen jeweils gleicher Segmente der M Probanden miteinander verglichen mit dem Ziel festzustellen, ob diesen M Probanden, die ein bestimmtes Organmerkmal aufweisen, auch eine bestimmte Veränderung oder typische Segmentstruktur der Iris zuordenbar ist, die sich beispielsweise über mehrere benachbarte Segmente der Iris in bestimmter Form, Farbgebung oder Kontrast erstrecken kann. Sofern sich eine solche eindeutige Zuordnung finden lässt, wird davon ausgegangen, dass dieses spezifische Organmerkmal eben genau in diesen aufgefundenen Segmenten oder Segmentstrukturen der Iris wiedergespiegelt wird. Auch von diesen M Probanden werden die Irisdaten gewonnwn und in einer weiteren "Norm-Iriskarte" zusammengefasst.

In einem weiteren Schritt werden die gewonnenen Dateien zur weiteren Bearbeitung aufbereitet dahingehend, dass eine "Standardform" oder Normalform dieser spezifischen Segmentkombination gebildet wird mit vorgegebener Abweichung hinsichtlich räumlicher Ausdehnung und/oder farblicher Gestaltung und/oder Intensität, so dass davon ausgegangen werden kann, dass bei der Eingabe eines Datensatzes mit einer Irisabbildung eines Probanden X mit unbekannten Organmerkmalen beim Durchlauf des Datensatzes eine wiederkehrende Segmentstruktur von dem die "Standardform" mit genannten Abweichungsbereichen enthaltenden Bearbeitungsprogramm eindeutig erkannt wird.

Mit der erfindungsgemässen Datenbank sind die Voraussetzungen geschaffen, um mit einer geeigneten Einrichtung, bestehend aus Scanner, Rechner und Rechenprogramm die Irisdaten eines beliebigen Probanden auf Übereinstimmung mit einer oder mehreren vorgegebenen Irisstruktur(en) zu vergleichen, die ihrerseits, wie oben beschrieben, einem bestimmten Organzustand entspricht.

Ein hierfür geeignetes Gerät mit der entsprechenden Software, die im wesentlichen die Irisdatenbank und ein geeignetes Auswahl- oder Filterprogramm enthält, ist einfach und ohne wesentliche medizinische Vorkenntnisse zu bedienen, da zwischen dem Eintrittsfenster des Scanners, beispielsweise eines Scanners mit der üblichen CCD-Technik, und dem Auge des Probanden eine vorgegebene räumliche Definition eingehalten werden muss, die ggf. durch entsprechende Masken oder Blenden fixiert werden kann.

Eine daraufhin durchgeführte Messung erlaubt in kürzester Zeit zumindest eine qualitative Aussage darüber, ob aus der Gesamtirisstruktur des Probanden sich auf eventuell nicht dem Normalzustand entsprechende Befindlichkeiten eines oder mehrerer Organe Rückschlüsse ziehen lassen.

Wenn die erforderliche Software in Form der Datenbank erstellt ist, ist die Durchführung des Verfahrens mit handelsüblichen Elektronikkomponenten, insbesondere PC's mit Datenbank-Software, problemlos möglich und erfordert nur geringe Investitionen.

### Kurze Beschreibung der Zeichnungen

Die einzelnen Verfahrensschritte werden nun anhand von Figuren näher erläutert; es zeigen:
- Figur 1:: Eine schematische Darstellung der Zuordnung von Bildpunkten zur als Ring dargestellten Iris zur Gewinnung einer Normdatei DQ,
- Figur 2:: eine Figur 1 entsprechende Darstellung mit auf Durchschnittswerte normierten Bildpunktwerten zur Gewinnung einer für ein Organ K spezifischen Durchschnittsdatei DQK,
- Figur 3:: eine entsprechende Darstellung mit Bildpunktwerten, gebildet aus der Differenz der Bildpunktwerte von entsprechenden Bildpunkten der Dateien DQ und DQK zur Gewinnung einer Differenzdatei M(Q-QK),und
- Figur 4:: schematische Darstellungen eines Polarkoordinatensystems zur Aufteilung der Bildpunktwerte.

### Beschreibung des Ausführungsbeispiels

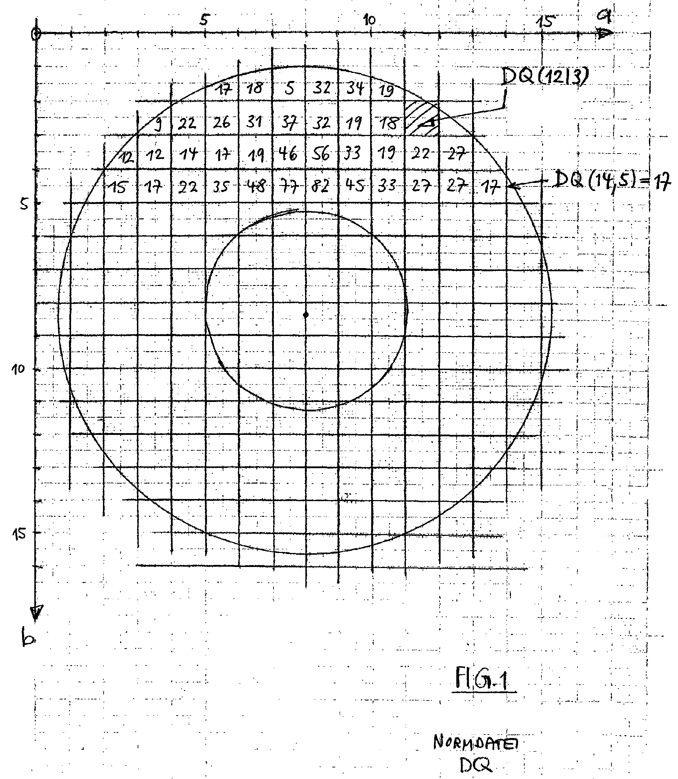

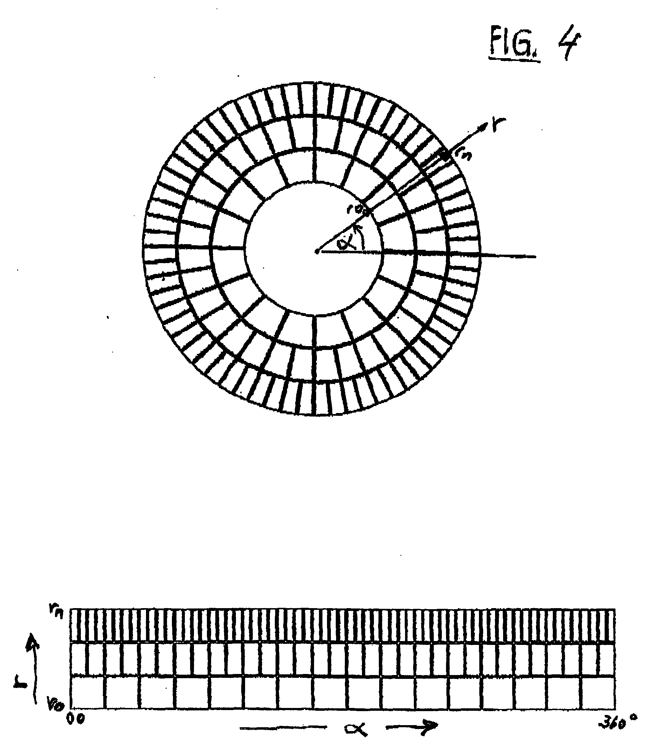

Figur 1 zeigt in schematischer Darstellung eine Iris eines Probanden M, die als Ring dargestellt ist (siehe Figur 4). Um eine elektronisch auswertbare Abbildung dieser Iris ("Iriskarte") zu gewinnen, wird ein Bildpunktraster über diese Iris gelegt, das beim dargestellten Ausführungsbeispiel aus Gründen der Übersichtlichkeit auf 16 x 16 Bildpunkte in einem kartesischen Koordinatensystem a,b beschränkt worden ist. Jedem optischen Bildpunkt wird ein entsprechendes Datenfeld zugeordnet.

Jedes Quadrat dieses Rasters soll somit einen Bildpunkt darstellen mit den Koordinaten a,b mit einem gemessenenen Farbwert, der ebenfalls aus Gründen der Vereinfachung als ganze Zahl mit Werten zwischen 0 und 100 dargestellt ist.

In Figur 1 sind die Datenfelder der Normdatei MQ "ihren" Bildpunkten zugeordnet. Dem in Figur 1 schraffiert dargestellten "Bildpunkt" mit den Koordinaten a=12 und b= 3 entspricht somit ein Datenfeld DQ mit der Adresse (12,3). Beim Datenfeld DQ(14,5) beträgt der mittlere gemessene Farbwert 17.

Bei einer sehr viel größeren Anzahl von Bildpunkten und einer entsprechenden Erhöhung der Farbtiefe (beispielsweise 12 bis 24 Bit) lässt sich auf diese Art und Weise durch optisch-elektronisches Einscannen die gewünschte hochauflösende Iriskarte für die linke und die rechte Iris eines Probanden gewinnen.

Diese Bildpunkte sind durch das Einscannen somit in eine dem Probanden M zugeordnete Datei DM mit jeweils a x b Datenfeldern umgesetzt, deren Wert dem digitalisierten Farbwert des zugehörigen Bildpunkts entspricht. Die Datei DM stellt dann somit die elektronische Iriskarte des Probanden M dar.

Dieses Verfahren wird für alle M Probanden wiederholt, so dass sich insgesamt M Dateien DM auf dem ersten Abschnitt eines geeigneten Datenträgers befinden, bspw. der Festplatte eines PC.

Aus diesen M Dateien DM wird rechnerisch eine Datei DQ gebildet, deren Datenfelder jeweils den Mittelwert oder einen anderen Normierungswert Q (a,b,) der entsprechenden Datenfelder der M Dateien DM darstellt. Die M Dateien werden also derart normiert, dass sich eine Normdatei DQ aus den normierten Werten Q (a,b) ergibt. Diese Normdatei DQ mit den Werten der in Figur 1 schematisch dargestellten Datenfelder stellt somit die "elektronische Durchschnitts-lriskarte" der M Probanden dar. Je nach dem Wert von M (Anzahl der Probanden) lassen sich somit auch Informationen über die statistische Abweichung einzelner Datenfelder bzw. Bildpunkte gewinnen, die eine Aussage darüber erlauben, wie weit ein bestimmter Bildwert bzw. dessen zugehöriges Datenfeld in statistisch relevanter Weise von dem zugehörigen Datenfeld der Normdatei MQ abweicht.

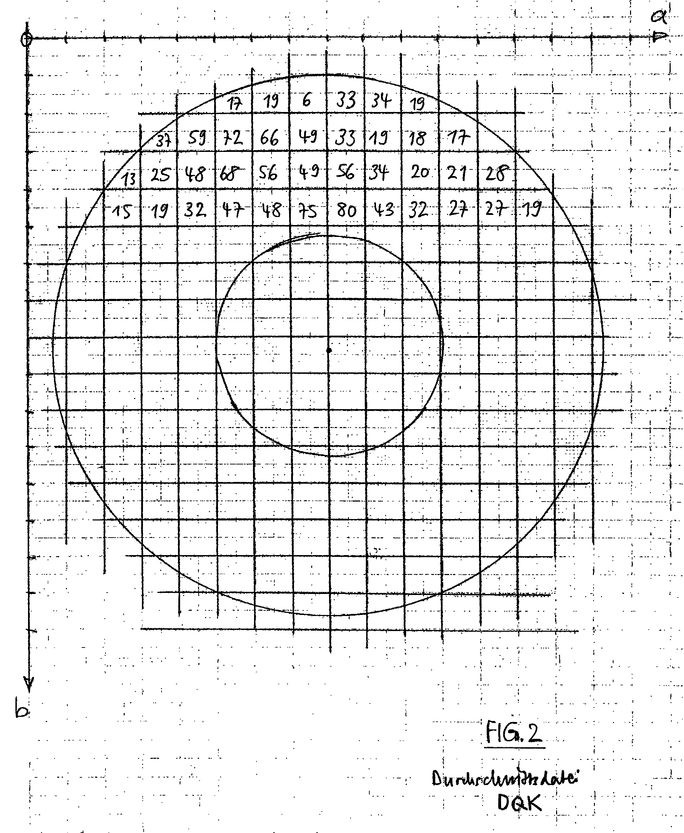

In weiterer Ausbildung des erfindungsgemäßen Verfahrens ist nun vorgesehen, dass N Probanden mit einem spezifischen, vom Normalzustand abweichenden Zustand jeweils eines Organs K ausgewählt werden. Das beschriebene Verfahren wird wiederholt, so dass eine Durchschnittsdatei DQK entsteht (Figur 2), die somit eine normierte Iriskarte bei einem bekannten, typischen Zustand des Organs K repräsentiert. Diese organ-spezifische Durchschnittsdatei wird auf einem zweiten Abschnitt des Datenträgers gespeichert.

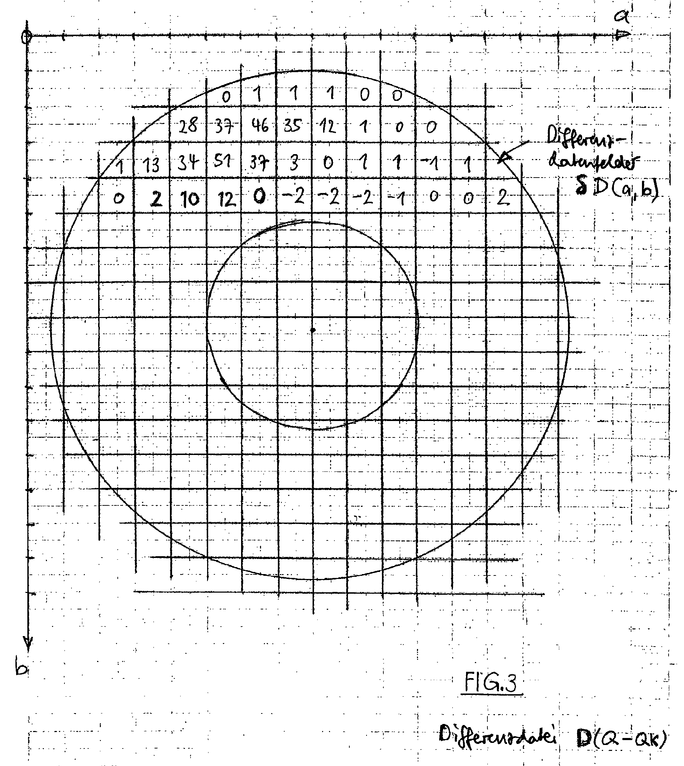

Als dritter Schritt ist vorgesehen, dass mittels einer Verknüpfung der Datenfelder, insbesondere einer Differenzbildung der Normdatei DQ und der Durchschnittdatei DQK eine Differenzdatei D (Q-QK) gebildet wird (Figur 3), deren Differenzdatenfelder δ D (a,b) aus der Differenz entsprechender Datenfelder der Normdatei MQ und der Durchschnittsdatei MQK gebildet sind.

Bei dem gewählten Zahlenbeispiel der Figuren 1 bis 3 kann man erkennen, dass bei einer unterstellten Abweichung des Zustandes des Organs K vom Normalzustand die Iris im linken oberen Segment eine signifikante Abweichung aufweist, oder anders ausgedrückt, die zugehörige elektronische Iriskarte weist in diesem Bereich stark abweichende Werte (entsprechend den Farbveränderungen der Iris) gegenüber der Norm-Iriskarte DQ auf.

Die auf einem dritten Abschnitt des Datenträgers abgespeicherte Information der Differenzdatenfelder zeigt somit auf, in welchem Bereich und in welchem Umfang die elektronische Iriskarte DQK bei einem bestimmten Zustand des Organs K typischerweise von der Norm-Iriskarte DK abweicht.

Mit dem ersten bis dritten Abschnitt des Datenträgers sind somit Informationen verfügbar, die typischen Organzuständen typische Abweichungen der zugehörigen Iriskarte von der Normal-Iriskarte zuweist, was die Möglichkeit eröffnet, bei einem Probanden X mit unbekanntem Zustand eines oder mehrerer Organe K durch Einscannen von dessen Iriskarte und Vergleich mit der Durchschnittsdatei DQK und/oder mit den K Differenzdateien gemäß Figur 3 (für jedes Organ K) festzustellen, ob in statistisch signifikanter Weise eine Übereinstimmung besteht, d.h., ob sich ein Organ K des Probanden X in einem von seinem Normalzustand abweichenden Zustand befindet, der durch eine typische Änderung seiner Iriskarte repräsentiert wird.

Beispielsweise wird dies dadurch erreicht, dass für entsprechende Bildpunkte a,b die Dateien für den Probanden X sukzessive mit dem im dritten Abschnitt des Datenträgers für die Organe K abgespeicherten Differenzdateien D(Q-QK) verglichen wird. Bei einem festsetzbaren Schwellenwert der Übereinstimmung oder Abweichung kann dies signalisiert werden, woraus sich dann die besagten Rückschlüsse auf den Organzustand ziehen lassen.

Bei den Figuren 1 bis 3 wurden kartesische Koordinaten a,b zur Gewinnung des Bildrasters für die Iriskarte benutzt, es ist jedoch genau so gut möglich, hierfür Polarkoordinaten (r,α) vorzusehen, wie dies in Figur 4 schematisch angedeutet ist. Der untere Teil stellt hierbei die Abwicklung der Darstellung des oberen Teils dar.

Die Polarkoordinaten haben den Vorzug, dass sie der kreisringförmigen Struktur der Iris entsprechen, so dass die räumliche Zuordnung der Datenfelder der entsprechenden Dateien zur Iriskarte anschaulicher wird. Auf die elektronische Speicherung und Auswertung hat dies jedoch keinen Einfluss, da sich die Koordinaten bei Bedarf rechnerisch ineinander überführen lassen und sich lediglich die Struktur der Dateien ändert, aber nicht deren Inhalt, der die Bildpunkte repräsentiert, unabhängig vom Format der Dateien.

## Patentansprüche

1. Verfahren zur Erstellung einer Datenbank mit Abbildungen der Iris von M Probanden, mit folgenden Verfahrensschritten:
(a) Gewinnung einer Abbildung der Iris (Iriskarte) der M Probanden durch optisch-elektronisches Einscannen der Bildpunkte mit bestimmtem Farbwert in einem vorgebbaren Raster.
(b) Umsetzung und Abspeicherung der M eingescannten Iriskarten in M Dateien mit jeweils a x b Datenfeldern mit einem Wert entsprechend dem Farbwert des zugehörigen Bildpunkts.
(c) Normierung der M Dateien derart, dass für jedes Datenfeld jeweils ein durch eine Rechenvorschrift vorgebbarer Normierungswert gebildet wird, der den normalen Farbwert des zugehörigen Bildpunktes darstellt.
(d) Bildung einer Normdatei (DQ) aus den derart normierten Mittelwerten, die eine Iriskarte mit die dem statistischen Normalzustand der Iris der M Probanden darstellt.
(e) Abspeicherung der Normdatei (DQ) in einem ersten Abschnitt eines Datenträgers.

2. Verfahren nach Anspruch 1, **dadurch gekennzeichnet, dass** es für N Probanden mit einem spezifischen, vom Normalzustand abweichenden Zustand jeweils eines Organs K wiederholt wird, woraus jeweils eine Durchschnittsdatei DQK entsteht, die eine Iriskarte mit dem statistischen Zustand der Iris bei einem spezifischen Zustand jeweils eines Organs K wiedergibt, und die in einem zweiten Abschnitt des Datenträgers abgespeichert werden.

3. Verfahren nach Anspruch 1 und 2, **dadurch gekennzeichnet, dass** durch Verknüpfung, insbesondere Differenzbildung einander entsprechender Datenfelder mit gleichem Wert von a und b der Normdatei (DQ) und einer Durchschnittsdatei (DQK) für jedes K jeweils eine Differenzdatei (D(Q-QK)) gebildet wird, deren über einem vorgegebenen Schwellenwert S liegender Wert einzelner oder mehrerer Differenzdatenfelder δD(a,b) einen statistisch signifikanten Indikator für einen vom Normalzustand abweichenden Zustand jeweils eines Organs K darstellt, und dass die Differenzdatei (D(Q-QK)) in einem dritten Abschnitt des Datenträgers abgespeichert wird.

4. Verfahren nach den vorhergehenden Ansprüchen, **dadurch gekennzeichnet, dass** es für einen Probanden X mit unbekanntem Organzustand wiederholt und auf einem vierten Abschnitt des Datenträgers abgespeichert wird.

5. Verfahren nach den vorhergehenden Ansprüchen, **dadurch gekennzeichnet, dass** einander entsprechende Datenfelder mit gleichen Werten für a und b des vierten Abschnitts des Datenträgers mit denen mindestens eines der anderen Abschnitte des Datenträgers verglichen werden, und das Vergleichsergebnis bei einer vorgebbaren Übereinstimmung oder Abweichung signalisiert wird.
